Europäisches Patentamt

European Patent Office

Office européen des brevets

(19)

(11) Veröffentlichungsnummer: **0 258 239**
**B1**

(12) **EUROPÄISCHE PATENTSCHRIFT**

(45) Veröffentlichungstag der Patentschrift: 25.07.90

(21) Anmeldenummer: 86902765.6

(22) Anmeldetag: 30.04.86

(86) Internationale Anmeldenummer:
PCT/AT86/00038

(87) Internationale Veröffentlichungsnummer:
WO 86/06267 06.11.86 Gazette 86/24

(51) Int. Cl.⁵: **A 61 B 6/00, A 61 B 6/04**

(54) VORRICHTUNG ZUR BEHANDLUNG VON GLIEDMASSEN.

(30) Priorität: 30.04.85 AT 1286/85

(43) Veröffentlichungstag der Anmeldung:
09.03.88 Patentblatt 88/10

(45) Bekanntmachung des Hinweises auf die
Patenterteilung:
25.07.90 Patentblatt 90/30

(84) Benannte Vertragsstaaten:
BE DE FR GB IT NL

(56) Entgegenhaltungen:
DE-A-2 640 248
FR-A-1 301 310
US-A-3 549 885
US-A-3 887 804
US-A-4 104 528
US-A-4 122 350
US-A-4 143 275

(73) Patentinhaber: ENDER, Hans Georg
Krenngasse 3
A-1180 Wien (AT)

(72) Erfinder: ENDER, Hans Georg
Krenngasse 3
A-1180 Wien (AT)

(74) Vertreter: Brauneiss, Leo et al
Patentanwälte Dipl.-Ing. Peter Boeckmann,
Dipl.-Ing. Leo Brauneiss Strohgasse 10
A-1030 Wien (AT)

**Beschreibung**

Die Erfindung betrifft eine Vorrichtung zur, insbesondere operativen Behandlung von Gliedmaßen, mit einer Auflageplatte für die zu behandelnde Gliedmaße, mit einem die Gliedmaße durchsetzende Strahlen oder Wellen aussendenden Sender zum Durchleuchten der zu behandelnden Gliedmaße, mit einem Empfänger für die Strahlen oder Wellen, und mit einem mit dem Empfänger gekuppelten Bildschirm zur Darstellung der zu behandelnden Gliedmaße während der Behandlung, wobei sich die Gliedmaße während der Behandlung etwa in der Mitte des Abstandes zwischen Sender und Empfänger befindet.

Bei der operativen Behandlung von Knochenbrüchen ist es bekannt, die Bruchstelle bei der Reposition, beim Einsetzen von Knochennägeln, beim Anlegen eines Gipsverbandes u.dgl. auf einem Bildschirm zu beobachten. Hiebei wird die zu behandelnde Gliedmaße auf einer Auflageplatte abgestützt, die von Röntgenstrahlen durchsetzt wird, welche von einer auf einer Seite der Auflageplatte angeordneten Röntgenröhre ausgesendet werden und zu einem Empfänger gelangen, der mit dem Bildschirm gekuppelt ist.

Bei den hiezu verwendeten, z.B. in der DE—AS—1 958 905 beschriebenen Geräten sind Röntgenröhre und Empfänger an den beiden Enden eines C-förmigen Trägers angeordnet. Diese Geräte werden so verwendet, daß die Auflageplatte unmittelbar auf dem Empfänger sitzt. Nachteilig ist bei diesen bekannten Geräten, daß die Abbildung auf dem Bildschirm verhältnismäßig klein ist und daß daher Details vom Operateur schlecht wahrgenommen werden können.

Auch bei andere beispielsweise aus der DE—OS—23 34 250 und aus der DE—OS—27 53 047 bekanntgewordenen Röntgenuntersuchungsgeräten ruht die Patientenauflagerstatt bzw. die Auflageplatte für den zu untersuchenden Körperteil entweder direkt am Empfänger auf oder ist in unmittelbarer Nachbarschaft zum Empfänger angeordnet.

Es wurde weiters bereits verschlagen (US—A—4 143 275) eine mittels Röntgenstrahlen hergestellte Abbildung auf einem Film dadurch zu vergrößern, daß der Film in Abstand vom abzubildenden Objekt angeordnet ist.

Die FR—A—1 301 310 offenbart eine Röntgeneinrichtung, bei welcher die Auflageplatte mittels Füßen auf einer Grundplatte abgestützt ist, wobei die Abstützung für die Filmkassette entlang der Füße verstellbar ist, somit gleichfalls in Abstand von der Auflageplatte angeordnet werden kann.

Schließlich zeigt die US—A—3 549 885 eine Röntgeneinrichtung, bei welcher auf einem kreisringförmigen Träger zwei Sender-Empfänger-Paare mit einander im wesentlichen senkrecht kreuzenden Strahlengängen vorgesehen sind, wobei die Empfänger mit einem Bildschirm gekuppelt sind. Die zu behandelnde Gliedmaße erstreckt sich durch den kreisringförmigen Träger etwa im Mittelpunkt desselben hindurch.

Die vorliegende Erfindung hat sich zur Aufgabe gestellt, eine bekannte, am Markt befindliche Vorrichtung zum Durchleuchten von Gliedmaßen mit einem Strahlen oder Wellen aussendenden Sender, einen Empfänger für diese Strahlen oder Wellen und einem mit dem Empfänger gekuppelten Bildschirm auf einfache Weise so umzugestalten, daß mit dieser Vorrichtung eine Vergrößerung der Abbildung auf dem Bildschirm ermöglicht wird, diese Vorrichtung jedoch auch in üblicher Weise für nicht vergrößerte Abbildungen herangezogen werden kann. Zur Lösung dieser Aufgabe wird vorgeschlagen, daß am Empfänger oder am Sender ein Gestell lösbar befestigt ist, welches die von den strahlen oder Wellen durchsetzbare Auflageplatte trägt. Bei Anordnung dieses Gestells mit der Auflageplatte befindet sich die zu behandelnde Gleidmaße in Abstand vom Empfänger, so daß hiedurch in bekannter Weise eine Vergrößerung der Abbildung erzielt wird. Diese Vergrößerung ist zwar mit einer Verringerung der Schärfe der Darstellung auf dem Bildschirm verbunden, es hat sich jedoch gezeigt, daß die entsprechende Unschärfe infolge der Bewegung der Gliedmaße während der Operation durch optische Aufarbeitung im Gehirn des Operateurs so umgewandelt wird, daß das Bild auf dem Bildschirm mit einer für die Operation völlig ausreichenden Deutlichkeit wahrgenommen wird. Dadurch, daß das die Auflageplatte tragende Gestell am Empfänger oder Sender befestigt ist, ist die Lage der Auflageplatte und damit der darauf abgestützten Gliedmaße und somit der Vergrößerungsmaßstab genau bestimmt. Ist eine Vergrößerung auf dem Bildschirm nicht erwünscht, so kann das Gestell mit der Auflageplatte ohne Schwierigkeiten auf einfache Weise entfernt und damit der ursprüngliche Zustand hergestellt werden, bei welchem sich beispielsweise die Auflageplatte direkt oberhalb des Empfängers befindet.

Zweckmäßig ist hiebei die Anordnung so getroffen, daß die Auflageplatte über, vorzugsweise an den vier Ecken derselben befestigte, Stützen mit einer beispielsweise aus Metall bestehenden Grundplatte verbunden ist, die am Empfänger oder am Sender abgestützt ist. Die Grundplatte kann hiebei mit einer Öffnung versehen sein, die in einen Gehäuseteil des Empfängers oder Senders eingesetzt ist. Diese Ausbildung ermöglicht auf einfache Weise die Befestigung der Auflageplatte am Empfänger oder Sender, wobei sichergestellt ist, daß die Auflageplatte die gewünschte Lage in Bezug auf den Empfänger oder Sender einnimmt.

Die Gehäuseteile, welche in die Öffnung der Grundplatte eingesetzt sind, weisen bei verschiedenen Fabrikaten der Durchleuchtungseinrichtung unterschiedliche Abmessungen innerhalb gewisser Grenzen auf. Um nun bei allen diesen Fabrikaten dieselbe Grundplatte verwenden zu können, ist gemäß einem weiteren Merkmal der Erfindung diese Grundplatte im Bereich ihrer Öffnung mit einem den Gehäuseteil des Empfängers oder Senders zumindest teilweise umschlingenden Spannband versehen. Dieses Spannband ermöglicht eine sichere Verankerung der Grundplatte am Gehäuseteil auch dann, wenn die Öffnung der Grundplatte größer ist als der in die Öffnung eingesetzte Gehäuseteil. Damit das Spannband über einen Großteil seiner Länge eng am Gehäuseteil anliegt, sind gemäß einem weiteren Merkmal der Erfindung die Enden des Spannbandes mit Gleitstücken verbunden, die in in Richtung zur Mitte der Öffnung der Grundplatte verlaufenden Führungen verschiebbar angeordnet sind. Durch Verschiebung der Gleitstücke in den Führungen kann die Lage des Spannbandes auch im Bereich der Enden desselben an die Abmessungen des Gehäuseteiles angepaßt werden.

Häufig ist es erforderlich, die Situation auch auf einem Film festzuhalten, beispielsweise die operierte Gliedmaße nach der Reposition eines Bruches und dem Anlegen eines Gipsverbandes abzubilden. Um dies zu ermöglichen, kann erfindungsgemäß in der Grundplatte ein Einschub für eine Filmkassette vorgesehen sein. In diesem Fall muß die Grundplatte mit dem Empfänger verbunden werden, ist also in unmittelbarer Nachbarschaft des Empfängers angeordnet, so daß auch auf dem Film ebenso wie am Bildschirm die Darstellung der Gliedmaße vergrößert erfolgt.

Zweckmäßig besteht die Auflageplatte aus zwei parallel zur Auflagefläche verlaufenden Schichten, von welchen die eine aus strahlendurchlässigem Material und die andere aus strahlenundurchlässigem Material besteht, wobei die Schicht aus strahlenundurchlässigem Material mit einer kreisförmigen Öffnung versehen ist, deren Durchmesser im wesentlichen dem Durchmesser des Strahlenkegels an der Stelle der Auflageplatte entspricht. Hierdurch werden Streustrahlungen unterhalb der Auflageplatte vermieden und sichergestellt, daß lediglich die durch die kreisförmigen Öffnung in der Schicht aus strahlenundurchlässigem Material hindurchtretenden Strahlen zum Empfänger gelangen.

Eine Verhinderung des Austrittes einer Streustrahlung kann auch dadurch erfolgen, daß der Zwischenraum zwischen der Auflageplatte und der Grundplatte durch einen Strahlenschutzmantel abgeschirmt ist.

In der Zeichnung ist die Erfindung an Hand von Ausführungsbeispielen schematisch dargestellt. Fig. 1 zeigt die Gesamtanordnung der erfindungsgemäßen Vorrichtung, und zwar bei ihrem Einsatz zur operativen Behandlung der Finger eines Patienten. Fig. 2 stellt einen Teil der erfindungsgemäßen Vorrichtung, teilweise im Schnitt, teilweise in Ansicht, dar. Fig. 3 zeigt das Spannband für die Befestigung der Grundplatte am Empfänger. Fig. 4 zeigt in perspektiver Darstellung eine abgewandelte Ausführungsform eines Teiles der erfindungsgemäßen Vorrichtung.

Die Erfindungsgemäße Vorrichtung weist einen Ständer 1 mit einer lotrechten Säule 2 auf, an der eine Führung 3 in Richtung des Pfeiles 4 verschiebbar und in der eingestellten Lage fixierbar angeordnet ist. In der Führung 3 ist eine Stütze 5 in Richtung des Pfeiles 6, also im wesentlichen in horizontaler Richtung, verschiebbar und fixierbar angeordnet, die mit einem C-förmigen Träger 7 verbunden ist. An einen Ende dieses Trägers 7 ist ein Strahlen aussendender Sender 8, in der Regel eine Röntgenröhre, befestigt, am anderen Ende des Trägers 7 ein Empfänger 9 für die vom Sender 8 ausgesandten Strahlen, der über eine Leitung 10 mit einem Bildschirm 11 gekuppelt ist. Der Strahlenkegel der vom Sender 8 ausgesandten Strahlen ist bei 12a angedeutet.

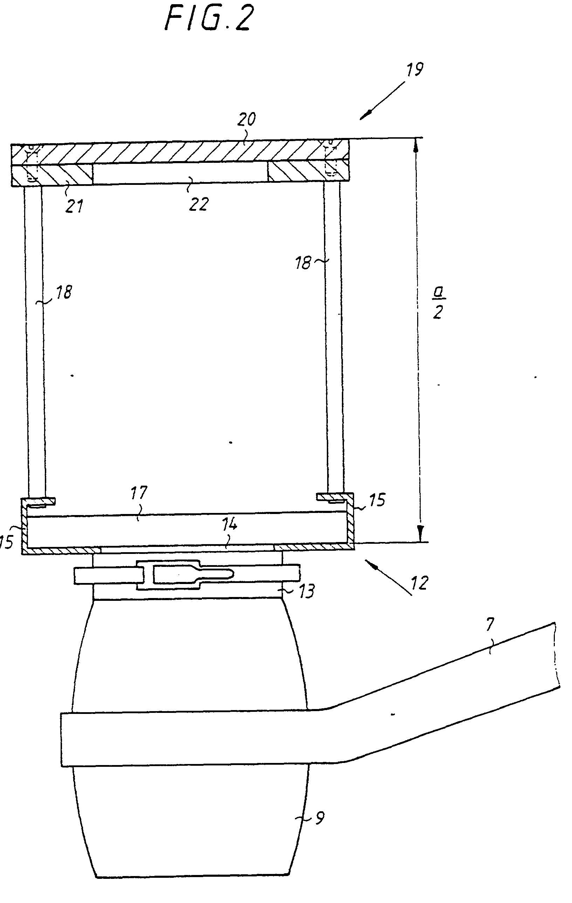

Wie auch aus Fig. 2 hervorgeht, ist der Empfänger 9 mit einer Grundplatte 12 verbunden, wobei ein Gehäuseteil 13 des Empfängers 9 im Bereich einer Öffnung 14 der Grundplatte 12 angeordnet ist. Die Grundplatte 12 weist an gegenüberliegenden Seiten C-förmige Führungen 15 auf, die einen Einschub für eine Filmkassette 16 bilden, der hinten durch einen Anschlag 17 begrenzt ist.

Die Grundplatte 12, welche aus Metall besteht, ist über vier Stützen 18 mit einer Auflageplatte 19 verbunden, die, wie insbesondere aus Fig. 2 hervorgeht, aus einer Schicht 20 aus strahlendurchlässigem Material und aus einer Schicht 21 aus strahlenundurchlässigem Material besteht, wobei die Schicht 21 mit einer kreisförmigen Öffnung 22 versehen ist, deren Durchmesser im wesentlichen dem Durchmesser des Strahlenkegels 12a im Bereich der Auflageplatte 19 entspricht.

Die Anordnung ist so getroffen, daß die Auflageplatte 19 den Abstand a zwischen der Strahlenaustrittsstelle des Senders 8 und der Strahleneintrittsstelle des Empfängers 9 halbiert.

Der zu behandelnde Patient liegt auf einer Liege 23 und hat seinen Arm auf einem Beistelltisch 24 abgestützt, wobei die erfindungsgemäße Vorrichtung so positioniert ist, daß die Auflageplatte 19 etwa in selber Höhe wie der Beistelltisch 24 angeordnet ist. Auf der Auflageplatte 19 befindet sich die zu operierende Hand des Patienten, welche bei der Operation vom Strahlenkegel 12a durchdrungen wird, so daß bei der Operation eine Darstellung dieser Hand auf dem Bildschirm 11 erfolgt. Dadurch, daß nun die Auflageplatte 19 in der Mitte des Abstandes a zwischen Sender und Empfänger angeordnet ist, also der Durchmesser des Strahlenkegels 12a im Bereich der Auflageplatte 19 halb so groß ist wie der Durchmesser dieses Strahlenkegels beim Auftreffen auf dem Empfänger 9, erfolgt eine Vergrößerung der Darstellung auf dem Bildschirm 11 auf den doppelten Wert. Auch bei Filmaufnahmen findet eine solche Vergrößerung statt, da sich der Einschub für die Filmkassette 16 gleichfalls im Bereich des Empfängers 9 befindet.

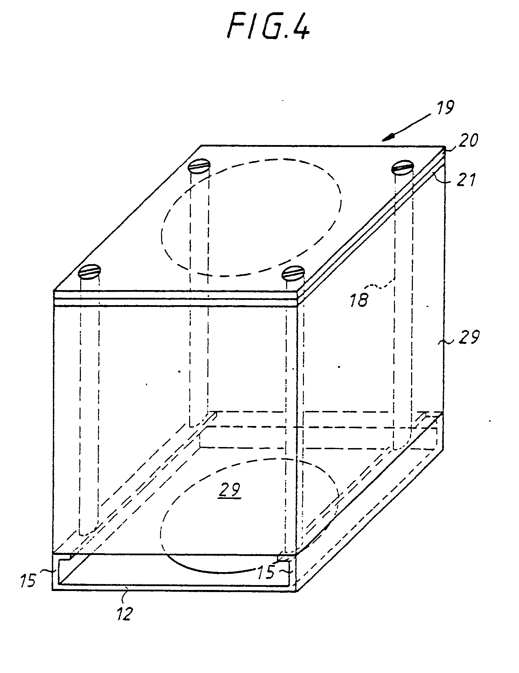

Fig. 3 zeigt die spezielle Anordnung der Befestigung der Grundplatte 12 am Gehäuseteil 13 des Empfängers 9. An der Unterseite der Grundplatte 12 sind radial zur Mitte der Öffnung 14 verlaufende Führungen 25 vorgesehen, in welchen Gleitstücke 26 verschiebbar geführt sind, die mit den Enden eines Spannbandes 27 verbunden sind, das den Gehäuseteil 13 des Empfängers 9 eng umschließt und mit einer bekannten Spannvorrichtung 28 versehen ist. Diese Ausführung ermöglicht eine sichere Verankerung der Grundplatte 12 an Empfängern 9, deren Gehäuseabmessungen innerhalb gewisser Grenzen variieren, wobei durch die in den Führungen 25 verschiebbar angeordneten Gleitstücke 26 sichergestellt ist, daß das Spannband 27 stets eng am Gehäuseteil 13 des Empfängers 8 anliegt.

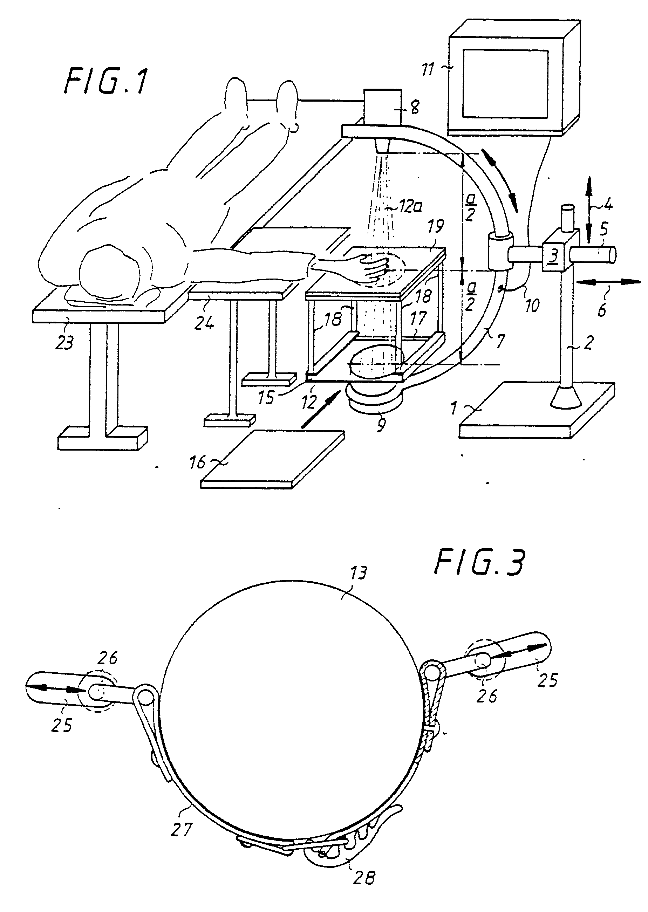

Die Ausführungsform nach Fig. 4 unterscheidet sich von der Ausführungsform nach den Fig. 1 bis 3 dadurch, daß im Bereich des Umfanges der Auflageplatte 19 sich in Richtung der Grundplatte 12 erstreckende und mit dieser Grundplatte 12 verbundene Wände 29 aus strhalenundurchlässigem Material vorgesehen sind, so daß der Zwischenraum zwischen Auflageplatte 19 und Grundplatte 12 durch einen Strahlenschutzmantel abgeschirmt ist.

**Patentansprüche**

1. Vorrichtung zur, insbesondere operativen, Behandlung von Gliedmaßen, mit einer Auflageplatte (19) für die zu behandelnde Gliedmaße, mit einem die Gliedmaße durchsetzende Strahlen oder Wellen aussendenden Sender (8) zum Durchleuchten der zu behandelnden Gliedmaße, mit einem Empfänger (9) für die Strahlen oder Wellen, und mit einem mit dem Empfänger (9) gekuppelten Bildschirm (11) zur Darstellung der zu behandelnden Gliedmaße während der Behandlung, wobei sich die Gliedmaße während der Behandlung etwa in der Mitte des Abstandes (a) zwischen Sender (8) und Empfänger (9) befindet, dadurch gekennzeichnet, daß am Empfänger (9) oder am Sender (8) ein Gestell (12, 18) lösbar befestigt ist, welches die von den strahlen oder Wellen durchsetzbare Auflageplatte (19) trägt.

2. Vorrichtung nach Anspruch 1, dadurch gekennzeichnet, daß die Auflageplatte (19) über, vorzugsweise an den vier Ecken derselben befestifte, Stützen (18) mit einer beispielsweise aus Metall bestehenden Grundplatte (12) verbunden ist, die am Empfänger (9) oder am Sender (8) abgestützt ist.

3. Vorrichtung nach Anspruch 1 und 2, dadurch gekennzeichnet, daß die Grundplatte (12) mit einer Öffnung (14) versehen ist, in die ein Gehäuseteil (13) des Empfängers (9) oder Senders (8) eingesetzt ist.

4. Vorrichtung nach einem der Ansprüche 1 bis 3, dadurch gekennzeichnet, daß die Grundplatte (12) im Bereich ihrer Öffnung (14) mit einem den Gehäuseteil (13) des Empfängers (9) oder Senders (8) zumindest teilweise umschlingenden Spannband (27) versehen ist.

5. Vorrichtung nach Anspruch 4, dadurch gekennzeichnet, daß die Enden des Spannbandes (27) mit Gleitstücken (26) verbunden sind, die in in Richtung zur Mitte der Öffnung (14) der Grundplatte (12) verlaufenden Führungen (25) verschiebbar angeordnet sind.

6. Vorrichtung nach einem der Ansprüche 1 bis 5, dadurch gekennzeichnet, daß in der Grundplatte (12) ein Einschub (15) für eine Filmkassette (16) vorgesehen ist.

7. Vorrichtung nach einem der Ansprüche 1 bis 6, dadurch gekennzeichnet, daß die Auflageplatte (19) aus zwei parallel zur Auflagefläche verlaufenden Schichten (20, 21) besteht, von welchen die eine (20) aus strahlendurchlässigem Material und die andere (21) aus strahlenundurchlässigem Material besteht, wobei die Schicht (21) aus strahlenundurchlässigem Material mit einer kreisförmigen Öffnung (22) versehen ist, deren Durchmesser im wesenltichen den Durchmesser des Strahlenkegels (12a) an der Stelle der Auflageplatte (19) entspricht.

8. Vorrichtung nach einem der Ansprüche 1 bis 7, dadurch gekennzeichnet, daß der Zwischenraum zwischen der Auflageplatte (19) und der Grundplatte (12) durch einen Strahlenschutzmantel (29) abgeschirmt ist (Fig. 4).

**Revendications**

1. Dispositif pour le traitement, en particulier chirurgical, de membres, comprenant une plaque d'appui (19) pour le membre à traiter, un émetteur (8) émettant un rayonnement ou des ondes qui traversent le membre en vue de l'examen radiographique du membre à traiter, un récepteur (9) pour le rayonnement ou les ondes, et un écran vidéo (11) accouplé au récepteur (9) pour la représentation du membre à traiter au cours du traitement, le membre se trouvant au cours du traitement sensiblement à la moitié de la distance (a) entre l'émetteur (8) et le récepteur (9), caractérisé par le fait qu'un bâti (12, 18) est fixé de manière amovible au récepteur (9) ou à l'émetteur (8) pour porter la plaque d'appui (19) qui peut être traversée par le rayonnement ou les ondes.

2. Dispositif selon la revendication 1, caractérisé par le fait que la plaque d'appui (19) est reliée par des montants (18), de préférence fixés aux quatre coins de celle-ci, à une plaque de base (12) qui est par exemple constituée de métal et qui s'appuie sur le récepteur (9) ou sur l'émetteur (8).

3. Dispositif selon la revendication 1 ou 2, caractérisé par le fait que la plaque de base (12) est munie d'une ouverture (14) dans laquelle est introduite une partie (13) du carter du récepteur (9) ou de l'émetteur (8).

4. Dispositif selon l'une des revendications 1 à 3, caractérisé par le fait que la plaque de base (12) est munie dans la région de son ouverture (14) d'une bande de serrage (27) qui entoure au moins partiellement la partie (13) du carter du récepteur (9) ou de l'émetteur (8).

5. Dispositif selon la revendication 4, caractérisé par le fait que les extrémités de la bande de serrage (27) sont reliées à des coulisseaux (26) qui sont montés en pouvant coulisser dans des guidages (25) s'étendant dans la direction du milieu de l'ouverture (14) de la plaque de base (12).

6. Dispositif selon l'une des revendications 1 à 5, caractérisé par le fait qu'un emboîtement (15) est prévu dans la plaque de base (12) pour l'insertion d'une cassette de film (16).

7. Dispositif selon l'une des revendications 1 à 6, caractérisé par le fait que la plaque d'appui (19) se compose de deux couches (20, 21) s'étendant parallèlement à la surface d'appui dont l'une (20) est constituée par un matériau transparent au rayonnement et dont l'autre (21) l'est en un matériau opaque au rayonnement, la couche (21) en un matériau opaque au rayonnement étant munie d'une ouverture circulaire (22) dont le diamètre correspond pour l'essentiel au diamètre du faisceau conique du rayonnement (12a) à l'endroit de la plaque d'appui (19).

8. Dispositif selon l'une des revendications 1 à 7, caractérisé par le fait que l'espace compris entre la plaque d'appui (19) et la plaque de base (12) est couvert par une enveloppe (29) protégeant des rayonnements (figure 4).

**Claims**

1. A device for treating limbs, in particular surgically, having a supporting plate (19) for the limb to be treated, having a transmitter (8) sending out rays or waves which penetrate the limb, for trans-illuminating the limb to be treated, having a receiver (9) for the rays or waves, and having a picture screen (11), coupled with the receiver (9), for showing the limb to be treated during treatment, the limb being, during treatment, approximately in the centre of the distance (a) between the transmitter (8) and the receiver (9), characterised in that a framework (12, 18), carrying the supporting plate (19) which can be penetrated by the rays or waves, is detachably fixed to the receiver (9) or the transmitter (8).

2. A device according to Claim 1, characterised in that the supporting plate (19) is connected, via posts (18) preferably fixed to four corners of the said plate, to a base plate (12) made for example of metal and supported against the receiver (9) or the transmitter (8).

3. A device according to Claim 1 and 2, characterised in that the base plate (12) is provided with an opening (14) into which a housing part (13) of the receiver (9) or of the transmitter (8) is inserted.

4. A device according to any one of Claims 1 to 3, characterised in that the base plate (12) is provided, in the region of its opening (14) with a tightening strap (27) embracing at least partly the housing part (13) of the receiver (9) or of the transmitter (8).

5. A device according to Claim 4, characterised in that the ends of the tightening strap (27) are connected to sliders (26) which are arranged in guides (25) extending towards the centre of the opening (14) in the base plate (12).

6. A device according to any one of Claims 1 to 5, characterised in that a drawer (15) is provided for a film cassette (16).

7. A device according to any one of Claims 1 to 6, characterised in that the supporting plate (19) comprises two layers (20, 21) extending parallel to the supporting face, one of which (20) is made of ray-permeable material and the other (21) of ray-impermeable material, the layer (21) of ray-impermeable material being provided with a circular opening (22) whose diameter is substantially the same as the diameter of the ray cone (12a) at the point of the supporting plate (19).

8. A device according to any one of Claims 1 to 7, characterised in that the gap between the supporting plate (19) and the base plate (12) is screened off by a ray-protective jacket (29) (Fig. 4).

**FIG.1**

**FIG.3**

## FIG.2

# FIG.4